# EUROPÄISCHE PATENTSCHRIFT

(11) **EP 1 656 184 B1**
(45) Veröffentlichungstag und Bekanntmachung des Hinweises auf die Patenterteilung: **26.03.2008**
(21) Anmeldenummer: 04764143.6
(22) Anmeldetag: 14.08.2004
(51) Int. Cl.: A61Q 5/04, A61Q 5/12, A61K 8/64, A61K 8/65, A61K 8/41, A61K 8/81, A61K 8/898, A61K 8/98, A61K 8/44

(54) **VERFAHREN ZUR GLÄTTUNG KERATINHALTIGER FASERN**
METHOD FOR SMOOTHING FIBRES CONTAINING KERATIN
PROCEDE POUR LISSER DES FIBRES KERATINIQUES

(30) Priorität: 23.08.2003 DE 10338883
(43) Veröffentlichungstag der Anmeldung: 17.05.2006
(73) Patentinhaber: HENKEL KOMMANDITGESELLSCHAFT AUF AKTIEN, 40589 Düsseldorf (DE)
(72) Erfinder: MÜLLER, Burkhard, 21075 Hamburg (DE); SCHELLIN, Aaltje, 21039 Hamburg (DE); NEUBÜSER, Inge, 22589 Hamburg (DE)
(74) Vertreter: Peters, Frank M.
(86) Internationale Anmeldenummer: PCT/EP2004/009151
(87) Internationale Veröffentlichungsnummer: WO 2005/020943

(56) Entgegenhaltungen:
- EP-A- 0 361 391
- WO-A-99/17719
- DE-A- 4 131 992
- GB-A- 2 114 616
- GB-A- 2 197 352
- US-A- 5 060 680
- US-A- 5 294 230
- US-A- 6 125 856
- US-A1- 2003 009 834
- HOLLENBERG D ET AL: "MODERNE STYLINGMITTEL-FUNKTION UND EIGENSCHAFTEN VON WELLMITTELN" SOFW-JOURNAL SEIFEN, OELE, FETTE, WACHSE, VERLAG FUR CHEMISCHE INDUSTRIE, H. ZIOLKOWSKY K.G. AUGSBURG, DE, Bd. 117, Nr. 3, 21. Februar 1991 (1991-02-21), Seiten 81-87, XP000201586 ISSN: 0942-7694
- DATABASE WPI Derwent Publications Ltd., London, GB; AN 1985-065774 XP002308543 NN: "Straightening curly hair" & JP 60 021704 A (ROKUJUDO KK) 4. Februar 1985 (1985-02-04)
- PATENT ABSTRACTS OF JAPAN Bd. 1997, Nr. 09, 30. September 1997 (1997-09-30) & JP 09 132515 A (MAEKAWA SEIJI), 20. Mai 1997 (1997-05-20)
- ALEXANDER P.: "Permanent Waving" MANUFACTURING CHEMIST, Bd. 59, Nr. 5, 1988, Seiten 61-64, XP002118531 GB

## Beschreibung

Die Erfindung betrifft ein Verfahren zur Glättung keratinhaltlger Fasern, insbesondere menschlicher Haare, sowie die Verwendung eines Mittels, enthaltend mindestens eine konditionierend wirkende Verbindung, ausgewählt aus kationischen Polymeren, quaternären Ammoniumverbindungen, Silikonen und Proteinhydrolysaten, in diesem Verfahren zur Glättung keratinhaltiger Fasern.

Als keratinhaltige Fasern können prinzipiell alle tierischen Haare, z.B. Wolle, Roßhaar, Angorahaar, Pelze, Federn und daraus gefertigte Produkte oder Textilien eingesetzt werden. Bevorzugt wird die Erfindung jedoch im Rahmen einer Glättung krauser menschlicher Haare und daraus gefertigter Perücken eingesetzt.

Eine dauerhafte Verformung keratinhaltiger Fasern wird üblicherweise derart durchgeführt, daß man die Faser mechanisch verformt und die Verformung durch geeignete Hilfsmittel festlegt. Vor und/oder nach dieser Verformung behandelt man die Faser mit einer keratinreduzierenden Zubereitung. Nach einem Spülvorgang wird die Faser dann mit einer Oxidationsmittelzubereitung behandelt, gespült und von den Verformungshilfsmitteln (Wicklern, Papilloten) befreit. Wenn als keratinreduzierende Komponente ein Merkaptan, z.B. Ammoniumthioglykolat, verwendet wird, spaltet dieses einen Teil der Disulfid-Brücken des Keratin-Moleküls zu -SH-Gruppen, so daß es zu einer Erweichung der Keratinfaser kommt. Bei der späteren oxidativen Fixierung werden erneut Disulfid-Brücken im Haarkeratin geknüpft, so daß das Keratingefüge in der vorgegebenen Verformung fixiert wird. Alternativ ist es bekannt, zur Haarverformung anstelle der Merkaptane Sulfit zu verwenden. Durch Hydrogensulfit-Lösungen und/oder Sulfit-Lösungen und/oder Disulfit-Lösungen werden Disulfid-Brücken des Keratins in einer Sulfitolyse nach der Gleichung

R-S-S-R +HSO₃⁽⁻⁾ → R-SH +R-S-SO₃⁽⁻⁾

gespalten und auf diese Weise eine Erweichung der Keratinfaser erreicht. Hydrogensulfit-, sulfit- bzw. disulfithaltige Reduktionsmittel weisen nicht den starken Eigengeruch der merkaptanhaltigen Mittel auf. Die Spaltung kann wie zuvor geschildert in einem Fixierschritt mit Hilfe eines Oxidationsmittels unter Bildung von neuen Disulfid-Brücken wieder rückgängig gemacht werden.

Die permanente Glättung keratinhaltiger Fasern wird analog durch den Einsatz von keratinreduzierenden und -oxidierenden Zusammensetzungen erzielt. In einem entsprechenden Verfahren wird das krause Haar entweder auf Wickler mit einem großen Durchmesser von üblicherweise mehr als 15 mm gewickelt oder das Haar unter Einwirkung der keratinreduzierenden Zusammensetzung glattgekämmt. Eine weitere Möglichkeit der Haarglättung ist das Glätten mit einem heißen Eisen. Allerdings verändert sich die Struktur der keratinhaltigen Faser bei der Wärmebehandlung des Haars während des Glättens (siehe dazu R. McMullen et al., J. Cosmet. Sci., 1998, 49, 223-244). Dieser Änderung der Faserstruktur sollte durch geeignete Maßnahmen entgegengewirkt werden.

Gemäß JP-A-60 21704 ist es vorteilhaft, wenn vor der Wärmebehandlung mit heißen Eisen eine Creme auf Ölbasis auf das Haar aufgetragen wurde. Die Creme schützt die Struktur der keratinhaltigen Faser während der Wärmebehandlung und gewährleistet eine gleichmäßige Glättung. Die Verwendung von kationischen Polymeren, Silikonen, quaternären Ammoniumverbindungen und Proteinhydrolysaten wird in diesem Dokument nicht erwähnt.

Aus den Druckschriften EP-A1-1 099 391 und US 6,125,856 sind dem Fachmann Verfahren zur Haarglättung und -pflege bekannt, in welchem die Fasern nach der reduktiven Aufspaltung der Disulfidbrücken mit einem heißen Eisen wärmebehandelt bzw. gebügelt werden. Vor der Anwendung der heißen Eisen wird ein polypeptidhaltiges Konditioniermittel auf das Haar aufgetragen und auf dem Haar belassen. Die chemische Reaktion der polymeren Proteinmoleküle mit der Haarfaser während des Glättens schützt die Haarfaser vor der Hitzeeinwirkung (siehe auch R. McMullen et al., J. Cosmet. Sci., 1998, 49, 245-254) und wirkt der Strukturänderung der Haarfaser entgegen. Allerdings ist die Verwendung von Konditioniermitteln in dem oben genannten Verfahren mit einem Nachteil behaftet. Durch die Hitzebehandlung der Fasern in Gegenwart von Konditioniermitteln, insbesondere von konditionierend wirkenden Polymeren, z.B. auf Basis von Polyacrylsäure und ihren Derivaten, verkleben die Haarfasem während des Glättens zu Haarbündeln. Diese Verklebungen können irreversibel sein und somit dauerhaft den Griff, das Aussehen sowie die Frisierbarkeit des Haars negativ beeinflussen.

Des weiteren sind durch die Wärmebehandlung induzierte chemische Reaktionen des Konditioniermittels in Form von Zersetzung oder Umsetzung mit anderen Rezepturbestandteilen unerwünscht. Die konditionierendwirkenden Verbindungen verlieren dadurch einerseits ihre Wirkung und andererseits können die Reaktions- bzw. Zersetzungsprodukte physiologisch bedenklich sein.

Im allgemeinen haben die bekannten Glättungsverfahren mit Wärmebehandlung den weiteren Nachteil, daß sich die keratinhaltige Faser elektrostatisch auflädt. Darüber hinaus ist das Glättungsergebnis der bekannten Verfahren hinsichtlich des Glättungsgrades und der Gleichmäßigkeit der Glättung verbesserungswürdig.

Aufgabe der Erfindung ist es daher, ein Glättungsverfahren für keratinhaltige Fasern, insbesondere für menschliches Haar, bereitzustellen, das ein verbessertes Glättungsergebnis liefert und die Struktur der Haarfaser schont und somit das Haar pflegt.

Überraschenderweise wurde gefunden, daß die Aufgabe durch das unten näher erläuterte erfindungsgemäße Verfahren gelöst wird. In diesem erfindungsgemäßen Verfahren finden Zusammensetzungen Verwendung, die mindestens eine konditionierend wirkende Verbindung, ausgewählt aus kationischen Polymeren, quaternären Ammoniumverbindungen, Silikonen und Proteinhydrolysaten, enthalten.

In der Druckschrift WO-A1-93/05757 werden Fixiermittel für die dauerhafte Haarverformung offenbart, die neben einem Oxidationmittel mindestens ein kationisches Polymer sowie mindestens ein nichtionisches und mindestens ein amphoteres Tensid enthalten, wobei alle Bestandteile in einem bestimmten Mischungsverhältnis zueinander stehen. Unter einer Dauerverformung wird unter anderem eine Haarentkräuselung verstanden. Gemäß dieser Druckschrift wird eine Wärmebehandlung der Faser im Rahmen der Dauerverformung nicht offenbart.

In der Druckschrift US-A1-2003/0009834 werden lagerstabile oxidationsmittelhaltige Zusammensetzungen durch Zusatz einer Kombination aus mindestens einem kationischem Polymer, mindestens einem Fettalkohol, mindestens einem ethoxylierten Fettalkohol sowie mindestens einem Fettsäureamid, erhalten.

In der Druckschrift WO-A1-97/25964 werden leave-on Mittel zur Konditionierung keratinhaltiger Fasern offenbart, die kationische Polymere, insbesondere Polyquaternium-37, enthalten. Die Verwendung dieser Polymere In einem speziellen Verfahren zur Glättung keratinhaltiger Fasern wird nicht erwähnt.

Die Druckschrift AU-A-596928 betrifft Dauerwellmittel, die ein Proteinhydrolysat und ein Silikon enthalten. Die Mittel werden im Rahmen dieser Offenbarung stets in einem Verfahren zur Wellung des Haars verwendet. Eine Anwendung solcher Wellmittel in einem speziellen Verfahren zur Haarglättung wird an keiner Stelle genannt.

Ein erster Gegenstand der Erfindung ist ein Verfahren zur Glättung keratinhaltiger Fasern, insbesondere menschlicher Haare, in dem
(i) eine wäßrige Zusammensetzung (A) enthaltend mindestens eine keratinreduzierende Verbindung auf die Fasern aufgetragen wird,
(ii) die wäßrige Zusammensetzung (A) nach einer Einwirkzeit Z1 wieder abgespült wird, dann eine wäßrige Zusammensetzung (C), enthaltend mindestens eine konditionierend wirkende Verbindung, ausgewählt aus kationischen Polymeren, quaternären Ammoniumverbindungen, Silikonen und Proteinhydrolysaten, auf die Fasern aufgetragen wird und nach einer Einwirkzeit Z3 wieder abgespült wird,
(iii) die Fasern getrocknet und anschließend
(iv) die Fasern einer Wärmebehandlung unter mechanischer Glättung der Faser bei einer Temperatur von 120-220°C unterworfen werden,
(v) anschließend eine wäßrige Zusammensetzung (B), enthaltend mindestens ein Oxidationsmittel auf die Fasern aufgetragen und
(vi) nach einer Einwirkzeit Z2 wieder abgespült wird,
wobei die wäßrige Zusammensetzung (A) als konditionierend wirkende Verbindungen mindestens ein Proteinhydrolysat und mindestens ein Silikon und gegebenenfalls mindestens eine weitere konditionierend wirkende Verbindung, ausgewählt aus kationischen Polymeren und quaternären Ammoniumverbindungen enthält, und wobei die wäßrige Zusammensetzung (B) gegebenenfalls mindestens eine weitere konditionierend wirkende Verbindung, ausgewählt aus kationischen Polymeren, quaternären Ammoniumverbindungen, Silikonen und Proteinhydrolysaten, enthält.

Eine wäßrige Zusammensetzung im Sinne der Erfindung enthält mindestens 50 Gew.% Wasser bezogen auf das Gewicht der gesamten Zusammensetzung.

Unter einer mechanischen Glättung wird erfindungsgemäß eine Streckung der krausen Faser entlang ihrer längsten räumlichen Ausdehnung verstanden. Diese mechanische Glättung kann beispielsweise durch Kämmen oder Bürsten erfolgen.

Die Wärmebehandlung unter mechanischer Glättung der Faser findet bei einer Temperatur von 120-220°C, bevorzugt bei einer Temperatur von 140-200°C, statt. Die

Wärmebehandlung kann mit heißer Luft erfolgen. In diesem Fall, wird die Faser während des Kämmens genau an der Stelle erwärmt, an der die mechanische Glättung erfolgt. Darüber hinaus ist es besonders bevorzugt, daß die Wärmebehandlung nach Manier des Glättens mit Hilfe entsprechend temperierter Platten, insbesondere Metall- oder Keramikplatten, erfolgt, in dem die Platte auf die zu glättende Faser gedrückt wird und die an die Faser gedrückte Platte entlang der Faser bewegt wird. Die Platten können gegebenenfalls mit hitzebeständigen Werkstoffen beschichtet sein. Besonders bevorzugt wird die zu glättende keratinhaltige Faser zwischen zwei entsprechend temperierte Platten gepreßt und beide Platten zugleich entlang der längsten räumlichen Ausdehnung der Faser bewegt. Dabei ist es wiederum bevorzugt, daß beide Platten miteinander verbunden sind, so daß beide Platten gleichmäßig entlang der Faser bewegt werden können. Die Bewegung entlang der Faser findet entlang der längsten räumlichen Ausdehnung der Faser statt. Wird die Wärmebehandlung am lebenden Haar durchgeführt, so ist die Faser an einem Ende (Haarwurzel) befestigt. Die Platten werden in diesem Fall bevorzugt gleichmäßig von der Haarwurzel weg entlang der gesamten Faser bewegt. Durch diese Bewegung erfolgt eine mechanische Glättung der Faser. Ein entsprechendes Gerät zur Wärmebehandlung ist beispielsweise das Gerät "Ceramic Flat-Master" (Vertrieben durch: Efalock, Deutschland).

Eine trockene keratinhaltige Faser gemäß Schritt (iii) des erfindungsgemäßen Verfahrens liegt dann vor, wenn die den Haaren anhaftenden Wasserreste soweit verdampft sind, daß die Haare einzeln fallen. Bevorzugt ist bei einer trockenen keratinhaltigen Faser entweder der Feuchtigkeitsgehalt der Faser mit der Feuchtigkeit der Luft im wesentlichen im Gleichgewicht oder die Faser nimmt Feuchtigkeit aus der Luft der Umgebung auf. Eine solche trockene Faser wird bevorzugt durch Trocknung der nassen Faser mit heißer Luft unter Benutzung eines Föns erzielt.

In einer bevorzugten Ausführungsform der Erfindung werden die keratinhaltigen Fasern vor dem Schritt (i) angefeuchtet. Dies kann durch Besprühen der Fasern mit einer Flüssigkeit, bevorzugt mit Wasser, geschehen. Bevorzugterweise werden die Fasern vor Schritt (i) mit einem herkömmlichen Shampoo shampooniert, gespült und dann mit einem Handtuch frottiert. Nach Abschluß des Frottierschritts bleibt eine fühlbare Restfeuchtigkeit im Haar zurück.

Es ist bevorzugt, die Fasern direkt nach dem Schritt (i) und/oder während der Einwirkzeit Z1 aus Schritt (ii) und gegebenenfalls direkt vor Schritt (iv) mechanisch zu glätten.

Im erfindungsgemäßen Verfahren wird im Schritt (ii) nach der Einwirkzeit Z1 die wässrige Zusammensetzung (A) wieder abgespült, dann eine wäßrige Zusammensetzung (C), enthaltend mindestens eine konditionierend wirkende Verbindung, ausgewählt aus kationischen Polymeren, quaternären Ammoniumverbindungen, Silikonen und Proteinhydrolysaten, auf die Fasern aufgetragen und nach einer Einwirkzeit Z3 wieder abgespült. In einer bevorzugten Variante dieser Ausführungsform enthält die Zusammensetzung (C) zusätzlich ein kationisches Polymer. Desweiteren wird die Zusammensetzung (C) bevorzugt als eine Emulsion, insbesondere als O/W-Emulsion, formuliert. Zu diesem Zweck enthält die Zusammensetzung (C) bevorzugt die dazu üblichen Bestandteile, wie beispielsweise C₈-C₃₀-Fettalkohole und Emulgatoren. Erfindungsgemäße Emulgatoren werden weiter unten beschrieben.

Obwohl die Zusammensetzung (C) gründlich aus dem Haar gespült und das Haar vor dem Schritt (iv) getrocknet wurde, wirkt sich die Verwendung der Zusammensetzung (C) vorteilhaft auf das Verhalten der Fasern während der Wärmebehandlung aus. Wird die Wärmebehandlung unter Verwendung temperierter Metallplatten durchgeführt, wird durch die Vorbehandlung der Faser mit der Zusammensetzung (C) das Gleitvermögen der Metallplatten entlang der Haarfaser erhöht sowie die elektrostatische Aufladung der Haare herabgesetzt obwohl die Zusammensetzung (C) gründlich ausgespült wurde.

Die Einwirkzeit Z1 beträgt bevorzugt 5-60 Minuten, besonders bevorzugt 10-30 Minuten. Die Einwirkzeit Z2 beträgt bevorzugt 1-30 Minuten, besonders bevorzugt 5-20 Minuten. Die Einwirkzeit Z3 beträgt bevorzugt 1 Sekunde bis 60 Minuten, besonders bevorzugt 30 Sekunden bis 5 Minuten.

Die wäßrige Zusammensetzung (A) enthält als konditionierend wirkende Verbindungen mindestens ein Proteinhydrolysat und mindestens ein Silikon.

Weiterhin ist es erfindungsgemäß bevorzugt, wenn die wäßrige Zusammensetzung (B) als konditionierend wirkende Verbindung mindestens ein kationisches Polymer enthält.

Die in der wäßrigen Zusammensetzung (A) enthaltenen keratinreduzierenden Verbindungen werden bevorzugt ausgewählt aus Verbindungen mit mindestens einer Thiolgruppe sowie deren Derivate, aus Sulfiten, Hydrogensulfiten und Disulfiten.

Verbindungen mit mindestens einer Thiolgruppe sowie deren Derivate sind beispielsweise Thioglykolsäure, Thiomilchsäure, Thioäpfelsäure, Phenylthioglykolsäure, Mercaptoethansulfonsäure sowie deren Salze und Ester (wie z.B. Isooctylthioglycolat und Isopropylthioglycolat), Cysteamin, Cystein, Bunte Salze und Salze der schwefligen Säure. Bevorzugt geeignet sind die Monoethanolammoniumsalze- oder Ammoniumsalze der Thioglykolsäure und/oder der Thiomilchsäure sowie die freien Säuren. Diese werden in der Zusammensetzung (A) bevorzugt in Konzentrationen von 0,5 bis 2,0 mol/kg bei einem pH-Wert von 5 bis 12, insbesondere von 7 bis 9,5, eingesetzt. Zur Einstellung dieses pH-Wertes enthalten die erfindungsgemäßen Zusammensetzungen (A) üblicherweise Alkalisierungsmittel wie Ammoniak, Alkali- und Ammonium-carbonate und -hydrogencarbonate oder organische Amine wie Monoethanolamin.

Beispiele für keratinreduzierende Verbindungen der Disulfite, die in der Zusammensetzung (A) enthaltenen sein können, sind Alkalidisulfite, wie z.B. Natriumdisulfit (Na₂S₂O₅) und Kaliumdisulfit (K₂S₂O₅), sowie Magnesiumdisulfit und Ammoniumdisulfit ((NH₄)₂S₂O₅). Ammoniumdisulfit kann dabei erfindungsgemäß bevorzugt sein. Beispiele für keratinreduzierende Verbindungen der Hydrogensulfite, die in der Zusammensetzung (A) enthaltenen sein können, sind Hydrogensulfite als Alkali-, Magnesium-, Ammonium- oder Alkanolammonium-Salz auf Basis eines C₂-C₄-Mono-, Di- oder Trialkanolamins. Ammoniumhydrogensulfit kann dabei ein besonders bevorzugtes Hydrogensulfit sein. Beispiele für keratinreduzierende Verbindungen der Sulfite, die in der Zusammensetzung (A) enthalten sein können, sind Sulfite als Alkali-, Ammonium- oder Alkanolammonium-Salz auf Basis eines C₂-C₄-Mono-, Di- oder Trialkanolamins. Ammoniumsulfit ist dabei bevorzugt. Der pH-Wert der Zusammensetzung (A) wird bei Verwendung von Sulfit und/oder Disulfit und/oder Hydrogensulft bevorzugt auf einen Wert im Neutralbereich von pH 5 bis 8, bevorzugt von pH 6 bis 7.5 eingestellt.

Bevorzugte C₂-C₄-Alkanolamine sind erfindungsgemäß 2-Aminoethanol (Monoethanolamin) und N,N,N-Tris(2-hydroxyethyl)amin (Triethanolamin). Monoethanolamin ist ein besonders bevorzugtes C₂-C₄-Alkanolamin, das insbesondere in einer Menge von 0.2 bis 6 Gew.-% bezogen auf die gesamte Zusammensetzung (A), eingesetzt wird.

Die keratinreduzierende Verbindung wird bevorzugt in einer Menge von 5 bis 20 Gew.-%, bezogen auf die gesamte wäßrige Zusammensetzung (A), eingesetzt.

Darüberhinaus kann die Zusammensetzung (A) weitere Komponenten enthalten, die die Wirkung der keratinreduzierenden Verbindung auf das Keratin fördern. Solche Komponenten sind z.B. Quellmittel für keratinhaltige Fasern wie z.B. C₁-C₆-Alkohole und wasserlösliche Glykole oder Polyole wie z.B. Glycerin, 1,2-Propylenglykol oder Sorbit und Harnstoff oder Harnstoffderivate wie z.B. Allantoin und Guanidin sowie Imidazol und dessen Derivate. Eine bevorzugte weitere Komponenten ist 1,2-Propylenglykol, insbesondere in einer Menge von 0.1 bis 5 Gew.-%. Die Mengenangaben beziehen sich jeweils auf die gesamte Zusammensetzung (A). In einer bevorzugten Ausführung enthält die erfindungsgemäße Zusammensetzung (A) 0 bis 5 Gew.-% 1,2-Propylenglykol und/oder 0 bis 5 Gew.-% Harnstoff.

Erfindungsgemäße konditionierend wirkende Verbindungen vom Typ der quarternären Ammoniumverbindungen sind bevorzugt Ammoniumhalogenide, insbesondere Chloride und Bromide, wie Alkyltrimethylammoniumhalogenide, Dialkyldimethylammoniumhalogenide und Trialkylmethylammoniumhalogenide, z.B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27, Quaternium-83 und Quaternium-87 bekannten Imidazolium-Verbindungen. Die Alkylgruppen der oben genannten Verbindungen weisen bevorzugt 10 bis 18 Kohlenstoffatome auf.

Sogenannte Esterquats gehören ebenfalls zu den erfindungsgemäß bevorzugten quaternären Ammoniumverbindungen. Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex^{®}, Dehyquart^{®} und Armocare^{®} vertrieben. Die Produkte Armocare^{®} VGH-70, ein N,N-Bis(2-Palmitoyloxyethyl)dimethylammoniumchlorid, sowie Dehyquart^{®} F-75, Dehyquart^{®} C-4046, Dehyquart^{®} L80 und Dehyquart^{®} AU-35 sind Beispiele für solche Esterquats.

Proteinhydrolysate sind Produktgemische, die durch sauer, basisch oder enzymatisch katalysierten Abbau von Proteinen (Eiweißen) erhalten werden. Erfindungsgemäß können Proteinhydrolysate sowohl pflanzlichen als auch tierischen Ursprungs eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan^{®} (Cognis), Promois^{®} (Interorgana), Collapuron^{®} (Cognis), Nutrilan^{®} (Cognis), Gelita-Sol^{®} (Deutsche Gelatine Fabriken Stoess & Co), Lexein^{®} (Inolex) und Kerasol^{®} (Croda) vertrieben.

Ein bevorzugtes Proteinhydrolysat ist das Seidenproteinhydrolysat (Promois^{®} Silk 720, Promois^{®} Silk 1000), welches besonders bevorzugt als konditionierend wirkende Verbindung mindestens in der Zusammensetzung (A) enthalten ist.

Erfindungsgemäß ist ebenso die Verwendung von Proteinhydrolysaten pflanzlichen Ursprungs, z. B. Soja-, Mandel-, Reis-, Erbsen-, Kartoffel- und Weizenproteinhydrolysate. Solche Produkte sind beispielsweise unter den Warenzeichen Gluadin^{®} (Cognis), DiaMin^{®} (Diamalt), Lexein^{®} (Inolex) und Crotein^{®} (Croda) erhältlich.

Ebenfalls möglich ist der Einsatz von Derivaten der Proteinhydrolysate, beispielsweise in Form ihrer Fettsäure-Kondensationsprodukte. Solche Produkte werden beispielsweise unter den Bezeichnungen Lamepon^{®} (Cognis), Gluadin^{®} (Cognis), Lexein^{®} (Inolex), Crolastin^{®} (Croda) oder Crotein^{®} (Croda) vertrieben.

Wenngleich der Einsatz der Proteinhydrolysate als solche bevorzugt ist, können an deren Stelle gegebenenfalls auch anderweitig erhaltene Aminosäuregemische oder einzelne Aminosäuren und Aminosäurederivate wie beispielsweise Arginin, Asparagin, Asparaginsäure, Citrullin, Histidin, Omithin, Lysin und Pyrroglutaminsäure eingesetzt werden. Die Aminosäuren können sowohl als freie Aminosäure, als auch als Salze, z. B. als Hydrochloride oder der Alkali, Erdalkali oder Ammoniumsalze eingesetzt werden. Weiterhin haben sich auch Oligopeptide aus durchschnittlich 2-3 Aminosäuren, die einen hohen Anteil (> 50 %, insbesondere > 70 %) an den genannten Aminosäuren haben, als erfindungsgemäß einsetzbar erwiesen.

Erfindungsgemäß besonders bevorzugt sind Arginin, Asparagin, Asparaginsäure sowie deren Salze und Oligopeptide bzw. Hydrolysate, welche reich an den genannten bevorzugten Aminosäuren sind. Ganz besonders bevorzugt sind Asparagin und Asparaginsäure sowie deren Salze bzw. Hydrolysate.

In einer speziellen Ausführungsform ist die Zusammensetzung (C) frei von Proteinhydrolysaten.

Erfindungsgemäß verwendbare Silikone als konditionierend wirkende Verbindungen sind bevorzugt lineare, cyclische oder verzweigte Silikone ausgewählt aus den Typen der Cyclomethicone, Dimethiconole, Dimethiconcopolyole, Amodimethicone, Trimethylsilylamodimethicone und Phenyltrimethicone. Diese Silikontypen sind dem Fachmann unter der Nomenklatur der Cosmetic, Toiletry and Fragrance Association (CTFA) bekannt und in: M.D. Berthiaume, Society of the Cosmetic Chemists Monograph Series, "Silicones in Hair Care", Ed.: L. D. Rhein, Hrsg.: Society of the Cosmetic Chemists, 1997, Kapitel 2 offenbart, worauf an dieser Stelle explizit verwiesen wird. Polysiloxane, wie Dialkyl- und Alkylarylsiloxane, beispielsweise Dimethylpolysiloxan und Methylphenylpolysiloxan, sowie deren alkoxylierte Analoga, mit Hydroxylgruppen terminierte Analoga und quaternierte Analoga, sowie cyclische Siloxane. Dabei sind besonders die Silikone mit den INCI-Bezeichnungen Dimethicone, PEG-12 Dimethicone, PEG/PPG-18/18 Dimethicone, Cyclomethicone, Dimethiconol, Quaternium-80 und Amodimethicone sowie deren Gemische sind besonders bevorzugte Silikone.

Beispiele für solche Silikone sind die von Dow Corning unter den Bezeichnungen DC 190 (INCI-Bezeichnung: PEG/PPG-18/18 Dimethicone), DC 193 (INCI-Bezeichnung: PEG-12 Dimethicone), DC 200, DC1401 (INCI-Bezeichnung: Cyclomethicone, Dimethiconol) und DC 1403 (INCI-Bezeichnung: Dimethicone, Dimethiconol) vertriebenen Produkte sowie die Handelsprodukte DC 244 (INCI-Bezeichnung: Cyclomethicone), DC 344 (INCI-Bezeichnung: Cyclomethicone) und DC 345 (INCI-Bezeichnung: Cyclomethicone) von Dow Corning, Q2-7224 (Hersteller: Dow Corning; ein stabilisiertes Trimethylsilylamodimethicon, Dow Corning 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil Quat 3270 und 3272 (Hersteller: Th. Goldschmidt; diquaternäre Polydimethylsiloxane, INCI-Bezeichnung: Quatemium-80).

Unter kationischen Polymeren sind erfindungsgemäß Polymere zu verstehen, welche in der Haupt- und/oder Seitenkette eine Gruppe aufweisen, welche "temporär" oder "permanent" kationisch sein kann. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine C₁-C₄-Kohlenwasserstoffgruppe an eine aus Acrylsäure, Methacrylsäure oder deren Derivaten aufgebaute Polymerhauptkette gebunden sind, haben sich als besonders geeignet erwiesen.

Polymere, die mindestens ein Strukturelement der allgemeinen Formel (I) besitzen, 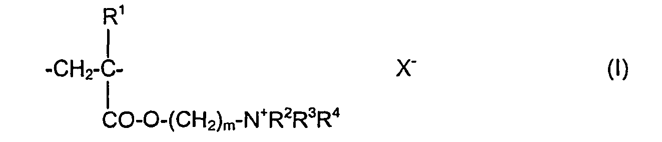 in der R¹ ein Wasserstofatom oder eine Methylgruppe ist, R², R³ und R⁴ unabhängig voneinander ausgewählt sind aus C₁-C₄-Alkyl-, C₁-C₄-Alkenyl- oder C₁-C₄-Hydroxyalkylgruppen, m = 1, 2, 3 oder 4 und X⁻ ein physiologisch verträgliches organisches oder anorganisches Anion ist, sind besonders bevorzugte kationische Polymere. Entsprechende Copolymere, bestehen im wesentlichen aus den in Formel (I) aufgeführten Strukturelementen sowie nichtionogenen Monomereinheiten. Homopolymere aus der Strukturelementen gemäß Formel (I) sind besonders bevorzugte kationische Polymere.

Im Rahmen dieser Polymere mit mindestens einer Struktureinheit gemäß Formel (I) sind diejenigen erfindungsgemäß ganz besonders bevorzugt, für die mindestens eine der folgenden Bedingungen gilt:
R¹ steht für eine Methylgruppe
R², R³ und R⁴ stehen für Methylgruppen
m hat den Wert 2.

Als physiologisch verträgliches Gegenionen X⁻ gemäß Formel (1) kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.

Ein besonders bevorzugtes Homopolymer ist das, gewünschtenfalls vernetzte, Poly(methacryloyloxyethyltrimethylammoniumchlorid) mit der INCI-Bezeichnung Polyquaternium-37. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vernetzungsagens.

Das Homopolymer wird bevorzugt in Form einer nichtwäßrigen Polymerdispersion, die einen Polymeranteil nicht unter 30 Gew.-% aufweisen sollte, eingesetzt. Solche Polymerdispersionen sind unter den Bezeichnungen Salcare^{®} SC 95 (ca. 50 % Polymeranteil, weitere Komponenten: Mineralöl (INCI-Bezeichnung: Mineral Oil) und Tridecyl-polyoxypropylen-polyoxyethylen-ether (INCI-Bezeichnung: PPG-1-Trideceth-6)) und Rheocare^{®} CTH (E) bzw. Salcare^{®} SC 96 (ca. 50 % Polymeranteil, weitere Komponenten: Mischung von Diestern des Propylenglykols mit einer Mischung aus Capryl- und Caprinsäure (INCI-Bezeichnung: Propylene Glycol Dicaprylate/Dicaprate) und Tridecylpolyoxypropylen-polyoxyethylen-ether (INCI-Bezeichnung: PPG-1-Trideceth-6)) im Handel erhältlich.

Copolymere mit Strukturelementen gemäß Formel (I) enthalten als nichtionogene Monomereinheiten bevorzugt Acrylamid, Methacrylamid, Acrylsäure-C₁₋₄-alkylester und Methacrylsäure-C₁₋₄-alkylester. Unter diesen nichtionogenen Monomeren ist das Acrylamid besonders bevorzugt. Auch diese Copolymere können, wie im Falle der Homopolymere oben beschrieben, vernetzt sein. Ein erfindungsgemäß bevorzugtes Copolymer ist das vernetzte Acrylamid-Methacryloyloxyethyltrimethylammoniumchlorid-Copolymer. Solche Copolymere, bei denen die Monomere in einem Gewichtsverhältnis von etwa 20:80 vorliegen, sind im Handel als ca. 50 %ige nichtwäßrige Polymerdispersion unter der Bezeichnung Salcare^{®} SC 92 erhältlich.

Weitere erfindungsgemäß verwendbaren kationischen Polymere sind beispielsweise
- quaternisierte Cellulose-Derivate, wie sie unter den Bezeichnungen Celquat^{®} und Polymer JR^{®} im Handel erhältlich sind. Die Verbindungen Celquat^{®} H 100, Celquat^{®} L 200 und Polymer JR^{®}400 sind bevorzugte quaternierte Cellulose-Derivate,
- kationische Alkylpolyglycoside gemäß der DE-PS 44 13 686,
- kationisierter Honig, beispielsweise das Handelsprodukt Honeyquat^{®} 50,
- kationische Guar-Derivate, wie insbesondere die unter den Handelsnamen Cosmedia^{®}Guar und Jaguar^{®} vertriebenen Produkte,
- Polysiloxane mit quaternären Gruppen, wie beispielsweise die im Handel erhältlichen Produkte Q2-7224 (Hersteller: Dow Corning; ein stabilisiertes Trimethylsilylamodimethicon), Dow Corning^{®} 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt), diquaternäre Polydimethylsiloxane, Quaternium-80),
- polymere Dimethyldiallylammoniumsalze und deren Copolymere mit Estern und Amiden von Acrylsäure und Methacrylsäure. Die unter den Bezeichnungen Merquat^{®} 100 (Poly(dimethyldiallylammoniumchlorid)) und Merquat^{®}550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat^{®}734 und Gafquat^{®}755 im Handel erhältlich,
- Vinylpyrrolidon-Vinylimidazoliummethochlorid-Copolymere, wie sie unter den Bezeichnungen Luviquat^{®} FC 370, FC 550, FC 905 und HM 552 angeboten werden,
- quaternierter Polyvinylalkohol,
- sowie die unter den Bezeichnungen Polyquaternium 2, Polyquaternium 17, Polyquaternium 18 und Polyquaternium 27 bekannten Polymeren mit quartären Stickstoffatomen in der Polymerhauptkette.

Gleichfalls als kationische Polymere eingesetzt werden können die unter den Bezeichnungen Polyquaternium-24 (Handelsprodukt z. B. Quatrisoft® LM 200), bekannten Polymere. Ebenfalls erfindungsgemäß verwendbar sind die Copolymere des Vinylpyrrolidons, wie sie als Handelsprodukte Copolymer 845 (Hersteller: ISP), Gaffix^{®} VC 713 (Hersteller: ISP), Gafquat^{®}ASCP 1011, Gafquat^{®}HS 110, Luviquat^{®}8155 und Luviquat^{®} MS 370 erhältlich sind.

Weitere erfindungsgemäße kationische Polymere sind die sogenannten "temporär kationischen" Polymere. Diese Polymere enthalten üblicherweise eine Aminogruppe, die bei bestimmten pH-Werten als quartäre Ammoniumgruppe und somit kationisch vorliegt. Bevorzugt sind beispielsweise Chitosan und dessen Derivate, wie sie beispielsweise unter den Handelsbezeichnungen Hydagen^{®} CMF, Hydagen^{®} HCMF, Kytamer^{®} PC und Chitolam^{®} NB/101 im Handel frei verfügbar sind.

Erfindungsgemäß bevorzugte kationische Polymere sind kationische Cellulose-Derivate und Chitosan und dessen Derivate, insbesondere die Handelsprodukte Polymer^{®} JR 400, Hydagen^{®} HCMF und Kytamer^{®} PC, kationische Guar-Derivate, kationische Honig-Derivate, insbesondere das Handelsprodukt Honeyquat^{®} 50, kationische Alkylpolyglycodside gemäß der DE-PS 44 13 686 und Polymere vom Typ Polyquaternium-37.

Es ist erfindungsgemäß bevorzugt, keine quaternisierten Proteinderivate als kationisches Polymer im Rahmen der vorliegenden Erfindung zu verwenden.

Die konditionierend wirkenden Verbindungen sind in den erfindungsgemäß verwendeten Zusammensetzungen bevorzugt in Mengen von 0,01 bis 10 Gew.-%, bezogen auf die jeweilige gesamte Zusammensetzung, enthalten. Mengen von 0,1 bis 5 Gew.-% sind besonders bevorzugt.

Weiterhin können die im erfindungsgemäßen Verfahren verwendeten Zusammensetzungen (A) und/oder (B) und/oder (C) mindestens einen oberflächenaktiven Stoff aus der Gruppe der anionischen, amphoteren, zwitterionischen und nichtionischen Tenside enthalten. Die Tenside haben unter anderem die Aufgabe, die Benetzung der Keratinoberfläche durch die Behandlungslösung zu fördern.

Als anionische Tenside eignen sich in erfindungsgemäßen Zubereitungen prinzipiell alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 bis 4 C-Atomen in der Alkanolgruppe,
- lineare und verzweigte Fettsäuren mit 8 bis 30 C-Atomen (Seifen),
- Ethercarbonsäuren der Formel R-O-(CH₂-CH₂O)ₓ-CH₂-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und-dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Alkylsulfate und Alkylpolyglykolethersulfate der Formel R-O(CH₂-CH₂O)ₓ-OSO₃H, in der R eine bevorzugt lineare Alkylgruppe mit 8 bis 30 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 8 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel (E1-I), 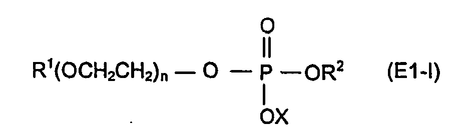 in der R¹ bevorzugt für einen aliphatischen Kohlenwasserstoffrest mit 8 bis 30 Kohlenstoffatomen, R² für Wasserstoff, einen Rest (CH₂CH₂O)ₙR¹ oder X, n für Zahlen von 1 bis 10 und X für Wasserstoff, ein Alkali- oder Erdalkalimetall oder NR³R ⁴R⁵R⁶, mit R³ bis R⁶ unabhängig voneinander stehend für Wasserstoff oder einen C1 bis C4 - Kohlenwasserstoffrest, steht,
- sulfatierte Fettsäurealkylenglykolester der Formel (E1-II)

   R⁷CO(AlkO)ₙSO₃M (E1-II)

   in der R⁷CO- für einen linearen oder verzweigten, aliphatischen, gesättigten und/oder ungesättigten Acylrest mit 6 bis 22 C-Atomen, Alk für CH₂CH₂, CHCH₃CH₂ und/oder CH₂CHCH₃, n für Zahlen von 0,5 bis 5 und M für ein Kation steht, wie sie in der DE-OS 197 36 906.5 beschrieben sind,
- Monoglyceridsulfate und Monoglyceridethersulfate der Formel (E1-III) 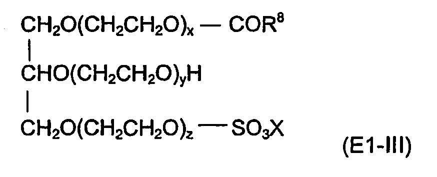 in der R⁸CO für einen linearen oder verzweigten Acylrest mit 6 bis 22 Kohlenstoffatomen, x, y und z in Summe für 0 oder für Zahlen von 1 bis 30, vorzugsweise 2 bis 10, und X für ein Alkali- oder Erdalkalimetall steht. Typische Beispiele für im Sinne der Erfindung geeignete Monoglycerid(ether)sulfate sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Talgfettsäuremonoglycerid sowie deren Ethylenoxidaddukte mit Schwefeltrioxid oder Chlorsulfonsäure in Form ihrer Natriumsalze. Vorzugsweise werden Monoglyceridsulfate der Formel (E1-III) eingesetzt, in der R⁸CO für einen linearen Acylrest mit 8 bis 18 Kohlenstoffatomen steht, wie sie beispielsweise in der EP-B1 0 561 825, der EP-B1 0 561 999, der DE-A1 42 04 700 oder von A.K.Biswas et al. in J.Am.Oil.Chem.Soc. 37, 171 (1960) und F.U.Ahmed in J.Am.Oil.Chem.Soc. 67, 8 (1990) beschrieben worden sind,
- Amidethercarbonsäuren wie sie in der EP 0 690 044 beschrieben sind,
- Kondensationsprodukte aus C₈ - C₃₀ - Fettalkoholen mit Proteinhydrolysaten und/oder Aminosäuren und deren Derivaten, welche dem Fachmann als Eiweissfettsäurekondensate bekannt sind, wie beispielsweise die Lamepon^{®} - Typen, Gluadin^{®} - Typen, Hostapon^{®} KCG oder die Amisoft^{®} - Typen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül, Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen, Monoglycerdisulfate, Alkyl- und Alkenyletherphosphate sowie Eiweissfettsäurekondensate.

Als zwitterionische Tenside werden solche oberflächenaktiven Verbindungen bezeichnet, die im Molekül mindestens eine quartäre Ammoniumgruppe und mindestens eine - COO⁽⁻⁾ - oder -SO₃⁽⁻⁾ -Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyldimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter ampholytischen Tensiden werden solche oberflächenaktiven Verbindungen verstanden, die außer einer C₈ - C₂₄ - Alkyl- oder -Acylgruppe im Molekül mindestens eine freie Aminogruppe und mindestens eine -COOH- oder -SO₃H-Grupp enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte ampholytische Tenside sind das N-Kokosalkylaminopropionat, das Kokosacylaminoethylaminopropionat und das C₁₂ - C₁₈ - Acylsarcosin.

Nichtionische Tenside enthalten als hydrophile Gruppe z.B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- mit einem Methyl- oder C₂ - C₆ - Alkylrest endgruppenverschlossene Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol^{®} LS, Dehydol^{®} LT (Cognis) erhältlichen Typen,
- C₁₂-C₃₀-Feftsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen® HSP (Cognis) oder Sovermol - Typen (Cognis),
- alkoxilierte Triglyceride,
- alkoxilierte Fettsäurealkylester der Formel (E4-I)

   R¹CO-(OCH₂CHR²)_{w}OR³ (E4-I)

   in der R¹CO für einen linearen oder verzweigten, gesättigten und/oder ungesättigten Acylrest mit 6 bis 22 Kohlenstoffatomen, R² für Wasserstoff oder Methyl, R³ für lineare oder verzweigte Alkylreste mit 1 bis 4 Kohlenstoffatomen und w für Zahlen von 1 bis 20 steht,
- Aminoxide,
- Hydroxymischether, wie sie beipielsweise in der DE-OS 19738866 beschrieben sind,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Zuckertenside vom Typ der Alkyl- und Alkenyloligoglykoside gemäß Formel (E4-II),

   R⁴O-[G]ₚ (E4-II)

   in der R⁴ für einen Alkyl- oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Stellvertretend für das umfangreiche Schrifttum sei hier auf die Übersichtsarbeit von Biermann et al. in Starch/Stärke 45, 281 (1993), B. Salka in Cosm.Toil. 108, 89 (1993) sowie J. Kahre et al. in SÖFW-Journal Heft 8, 598 (1995) verwiesen.
   Die Alkyl- und Alkenyloligoglykoside können sich von Aldosen bzw. Ketosen mit 5 oder 6 Kohlenstoffatomen, vorzugsweise von Glucose, ableiten. Die bevorzugten Alkyl- und/oder Alkenyloligoglykoside sind somit Alkyl- und/oder Alkenyloligoglucoside.
   Die Indexzahl p in der allgemeinen Formel (E4-II) gibt den Oligomerisierungsgrad (DP), d. h. die Verteilung von Mono- und Oligoglykosiden an und steht für eine Zahl zwischen 1 und 10. Während p im einzelnen Molekül stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt. Der Alkyl- bzw. Alkenylrest R⁴ kann sich von primären Alkoholen mit 4 bis 11, vorzugsweise 8 bis 10 Kohlenstoffatomen ableiten.
   Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R¹⁵ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3.
- Zuckertenside vom Typ der Fettsäure-N-alkylpolyhydroxyalkylamide, ein nichtionisches Tensid der Formel (E4-III), 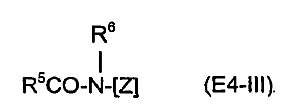 in der R⁵CO für einen aliphatischen Acylrest mit 6 bis 22 Kohlenstoffatomen, R⁶ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht. Bei den Fettsäure-N-alkylpolyhydroxyalkylamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können.
   Hinsichtlich der Verfahren zu ihrer Herstellung sei auf die US-Patentschriften US 1,985,424, US 2,016,962 und US 2,703,798 sowie die Internationale Patentanmeldung WO 92/06984 verwiesen. Eine Übersicht zu diesem Thema von H.Kelkenberg findet sich in Tens. Surf. Det. 25, 8 (1988). Vorzugsweise leiten sich die Fettsäure-N-alkylpolyhydroxyalkylamide von reduzierenden Zuckern mit 5 oder 6 Kohlenstoffatomen, insbesondere von der Glucose ab. Die bevorzugten Fettsäure-N-alkylpolyhydroxyalkylamide stellen daher Fettsäure-N-alkylglucamide dar, wie sie durch die Formel (E4-IV) wiedergegeben werden:

   R⁷CO-NR⁸-CH₂-(CHOH)₄CH₂OH (E4-IV)

   Vorzugsweise werden als Fettsäure-N-alkylpolyhydroxyalkylamide Glucamide der Formel (E4-IV) eingesetzt, in der R⁸ für Wasserstoff oder eine Alkylgruppe steht und R⁷CO für den Acylrest der Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Arachinsäure, Gadoleinsäure, Behensäure oder Erucasäure bzw. derer technischer Mischungen steht. Besonders bevorzugt sind Fettsäure-N-alkylglucamide der Formel (E4-IV), die durch reduktive Aminierung von Glucose mit Methylamin und anschließende Acylierung mit Laurinsäure oder C12/14-Kokosfettsäure bzw. einem entsprechenden Derivat erhalten werden. Weiterhin können sich die Polyhydroxyalkylamide auch von Maltose und Palatinose ableiten.

Als bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Diese Verbindungen sind durch die folgenden Parameter gekennzeichnet. Der Alkylrest R enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Weiterhin können als nichtionische Tenside die Zuckertenside in den Zusammensetzungen (A), (B) und/oder (C) enthalten sein. Diese können in den erfindungsgemäß verwendeten Zusammensetzungen bevorzugt in Mengen von 0,1 - 20 Gew.-%, bezogen auf die jeweilige gesamte Zusammensetzung, enthalten sein. Mengen von 0,5 - 15 Gew.-% sind bevorzugt, und ganz besonders bevorzugt sind Mengen von 0,5 - 7,5 Gew.%.

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Die Tenside werden in Mengen von 0,1 - 45 Gew.%, bevorzugt 0,5 - 30 Gew.% und ganz besonders bevorzugt von 0,5 - 25 Gew.%, bezogen auf die jeweilige gesamte erfindungsgemäß verwendete Zusammensetzung, eingesetzt.

In einer weiteren Ausführungsform werden in den wäßrigen Zubereitungen (A) und/oder (B) und/oder (C) des erfindungsgemäßen Verfahrens Emulgatoren verwendet. Emulgatoren bewirken an der Phasengrenzfläche die Ausbildung von wasser- bzw. ölstabilen Adsorptionsschichten, welche die dispergierten Tröpfchen gegen Koaleszenz schützen und damit die Emulsion stabilisieren. Emulgatoren sind daher wie Tenside aus einem hydrophoben und einem hydrophilen Molekülteil aufgebaut. Hydrophile Emulgatoren bilden bevorzugt O/W - Emulsionen und hydrophobe Emulgatoren bilden bevorzugt W/O - Emulsionen. Unter einer Emulsion ist eine tröpfchenförmige Verteilung (Dispersion) einer Flüssigkeit in einer anderen Flüssigkeit unter Aufwand von Energie zur Schaffung von stabilisierenden Phasengrenzflächen mittels Tensiden zu verstehen. Die Auswahl dieser emulgierenden Tenside oder Emulgatoren richtet sich dabei nach den zu dispergierenden Stoffen und der jeweiligen äußeren Phase sowie der Feinteiligkeit der Emulsion. Weiterführende Definitionen und Eigenschaften von Emulgatoren finden sich in "H.-D.Dörfler, Grenzflächen- und Kolloidchemie, VCH Verlagsgesellschaft mbH. Weinheim, 1994". Erfindungsgemäß verwendbare Emulgatoren sind beispielsweise
- Anlagerungsprodukte von 4 bis 100 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Polyole mit 3 bis 6 Kohlenstoffatomen, insbesondere an Glycerin,
- Ethylenoxid- und Polyglycerin-Anlagerungsprodukte an Methylglucosid-Fettsäureester, Fettsäurealkanolamide und Fettsäureglucamide,
- C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Fettalkoholen zum Beispiel das im Handel erhältliche Produkt Montanov^{®}68,
- Anlagerungsprodukte von 5 bis 60 Mol Ethylenoxid an Rizinusöl und gehärtetes Rizinusöl,
- Partialester von Polyolen mit 3-6 Kohlenstoffatomen mit gesättigten Fettsäuren mit 8 bis 22 C-Atomen,
- Sterine. Als Sterine wird eine Gruppe von Steroiden verstanden, die am C-Atom 3 des Steroid-Gerüstes eine Hydroxylgruppe tragen und sowohl aus tierischem Gewebe (Zoosterine) wie auch aus pflanzlichen Fetten (Phytosterine) isoliert werden. Beispiele für Zoosterine sind das Cholesterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.
- Phospholipide. Hierunter werden vor allem die Glucose-Phospolipide, die z.B. als Lecithine bzw. Phospahtidylcholine aus z.B. Eidotter oder Pflanzensamen (z.B. Sojabohnen) gewonnen werden, verstanden.
- Fettsäureester von Zuckern und Zuckeralkoholen, wie Sorbit,
- Polyglycerine und Polyglycerinderivate wie beispielsweise Polyglycerinpoly-12-hydroxystearat (Handelsprodukt Dehymuls^{®} PGPH),
- Lineare und verzweigte Fettsäuren mit 8 bis 30 C - Atomen und deren Na-, K-, Ammonium-, Ca-, Mg- und Zn - Salze.

Die erfindungsgemäßen Zusammensetzungen enthalten die Emulgatoren bevorzugt in Mengen von 0,1 - 25 Gew.-%, insbesondere 0,1 - 3 Gew.-%, bezogen auf die jeweilige gesamte Zusammensetzung.

Bevorzugt können die erfindungsgemäßen Zusammensetzungen (Ä) und/oder (B) und/oder (C) mindestens einen nichtionogenen Emulgator mit einem HLB-Wert von 8 bis 18, gemäß den im Römpp-Lexikon Chemie (Hrg. J. Falbe, M.Regitz), 10. Auflage, Georg Thieme Verlag Stuttgart, New York, (1997), Seite 1764, aufgeführten Definitionen enthalten. Nichtionogene Emulgatoren mit einem HLB-Wert von 10 - 15 können erfindungsgemäß besonders bevorzugt sein.

Die erfindungsgemäßen Zusammensetzungen (A) und/oder (B) und/oder (C) enthalten bevorzugt mindestens einen linearen oder verzweigten, gesättigten oder ungesättigten Fettalkohol. Als Fettalkohole können eingesetzt werden Fettalkohole mit C₆-C₃₀-, bevorzugt C₁₀-C₂₂- und ganz besonders bevorzugt C₁₂-C₂₂- Kohlenstoffatomen. Einsetzbar im Sinne der Erfindung sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Die Fettalkohole stammen jedoch von bevorzugt natürlichen Fettsäuren ab, wobei üblicherweise von einer Gewinnung aus den Estern der Fettsäuren durch Reduktion ausgegangen werden kann. Erfindungsgemäß einsetzbar sind ebenfalls solche Fettalkoholschnitte, die durch Reduktion natürlich vorkommender Triglyceride wie Rindertalg, Palmöl, Erdnußöl, Rüböl, Baumwollsaatöl, Sojaöl, Sonnenblumenöl und Leinöl oder aus deren Umesterungsprodukten mit entsprechenden Alkoholen entstehenden Fettsäureestern erzeugt werden, und somit ein Gemisch von unterschiedlichen Fettalkoholen darstellen. Solche Substanzen sind beispielsweise unter den Bezeichnungen Stenol^{®}, z.B. Stenol^{®} 1618 oder Lanette^{®}, z.B. Lanette^{®} O oder Lorol^{®}, z.B. Lorol^{®} C8, Lorol^{®} C14, Lorol^{®} C18, Lorol^{®} C8-18, HD-Ocenol^{®}, Crodacol^{®}, z.B. Crodacol^{®} CS, Novol^{®}, Eutanol^{®} G, Guerbitol^{®} 16, Guerbitol^{®} 18, Guerbitol^{®} 20, Isofol^{®} 12, Isofol^{®} 16, Isofol^{®} 24, Isofol^{®} 36, Isocarb^{®} 12, Isocarb^{®} 16 oder Isocarb^{®} 24 käuflich zu erwerben. Selbstverständlich können erfindungsgemäß auch Wollwachsalkohole, wie sie beispielsweise unter den Bezeichnungen Corona^{®}, White Swan^{®}, Coronet^{®} oder Fluilan^{®} käuflich zu erwerben sind, eingesetzt werden.

Die Fettalkohole werden in Mengen von 0,1 - 20 Gew.-%, bezogen auf die gesamte Zubereitung, bevorzugt in Mengen von 0,1 -10 Gew.-% eingesetzt.

Die erfindungsgemäße Zusammensetzung (A) und/oder (B) und/oder (C), insbesondere die Zusammensetzungen (A) und/oder (B), enthalten bevorzugt eine viskositätserhöhende Verbindung, im weiteren als Verdickungsmittel bezeichnet.

Erfindungsgemäß einsetzbare Verdickungsmittel sind beispielsweise Agar-Agar, Guar-Gum, Alginate, Xanthan-Gum, Gummi arabicum, Karaya-Gummi, Johannisbrotkernmehl, Leinsamengummen, Dextrane, Dicaprylate, Dicaprate, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol, sowie viskositätserhöhende Polymere auf Polyacrylat-Basis wie sie beispielsweise unter den Handelsnamen Pemulen^{®}, Aculyn^{®} und Carbopol^{®} vertrieben werden.

Die Zusammensetzung (A) besitzt bevorzugt eine Viskosität von 5000 bis 50000 mPa·s, insbesondere von 8000 bis 20000 mPa·s, bei 20°C (gemessen mit einem Brookfield-Viskometer, Spindel Nr. 6 bei 20 rpm).

Die Zusammensetzung (B) besitzt bevorzugt eine Viskosität von 1000 bis 30000 mPa·s, insbesondere 2000 bis 10000 mPa·s, bei 20 °C (gemessen mit einem Brookfield-Viskometer, Spindel Nr. 4 bei 20 rpm).

Das in der wäßrigen Zusammensetzung (B) enthaltene Oxidationsmittel wird bevorzugt ausgewählt aus z.B. Natriumbromat, Kaliumbromat oder Wasserstoffperoxid. Es ist besonders bevorzugt, Wasserstoffperoxid als Oxidationsmittel zu verwenden. Zur Stabilisierung wäßriger Wasserstoffperoxidzubereitungen können zusätzlich übliche Stabilisatoren zugesetzt werden. Der pH-Wert der wäßrigen H₂O₂-Zubereitungen, die üblicherweise etwa 0,5 bis 3,0 Gew.-% H₂O₂ enthalten, liegt bevorzugt bei 2 bis 6. Wäßrige Zusammensetzungen (B) auf Bromat-Basis enthalten die Bromate üblicherweise in Konzentrationen von 1 bis 10 Gew.-% und der pH-Wert der Lösungen wird auf 4 bis 7 eingestellt.
Oft werden Fixiermittel für die Dauerverformung keratinhaltiger Fasern als Feststoffe formuliert. Sie enthalten das Oxidationsmittel dann in Form eines Festkörpers, z.B. Natriumperborat. Erst kurz vor der Anwendung werden diese Mittel dann mit Wasser unter erhalt der wäßrigen Zusammensetzung (B) versetzt.

Weiterhin können folgende Verbindungen in den erfindungsgemäß verwendeten Zusammensetzungen (A) und/oder (B) und/oder (C) enthalten sein:
• lineare und/oder verzweigte Fettsäuren, bevorzugt C₂-C₃₀- Fettsäuren, besonders bevorzugt C₄-C₁₈ Fettsäuren, am meisten bevorzugt C₆-C₁₀-Fettsäuren und/oder deren physiologisch verträglichen Salzen; Weitere Beispiele sind Ameisensäure, Essigsäure, Propionsäure, Buttersäure, Isobuttersäure, Valeriansäure, Isovaleriansäure, Pivalinsäure, Oxalsäure, Malonsäure, Bernsteinsäure, Glutarsäure, Milchsäure, Glycerinsäure, Glyoxylsäure, Adipinsäure, Pimelinsäure, Korksäure, Azelainsäure, Sebacinsäure, Propiolsäure, Crotonsäure, Isocrotonsäure, Elaidinsäure, Maleinsäure, Fumarsäure, Muconsäure, Citraconsäure, Mesaconsäure, Camphersäure, Benzoesäure, o,m,p-Phthalsäure, Naphthoesäure, Toluoylsäure, Hydratropasäure, Atropasäure, Zimtsäure, Isonicotinsäure, Nicotinsäure, Bicarbaminsäure, 4,4'-Dicyano-6,6'-binicotinsäure, 8-Carbamoyloctansäure, 1,2,4-Pentantricarbonsäure, 2-Pyrrolcarbonsäure, 1,2,4,6,7-Napthalinpentaessigsäure, Malonaldehydsäure, 4-Hydroxy-phthalamidsäure, 1-Pyrazolcarbonsäure, Gallussäure oder Propantricarbonsäure,
• Polyhydroxyverbindungen; hierbei sind insbesondere zu nennen
   - Zucker mit 5 und/oder 6 Kohlenstoffatomen, insbesondere als Mono- und/oder Oligosaccharide, beispielsweise Glucose, Fructose, Galactose, Lactose, Arabinose, Ribose, Xylose, Lyxose, Allose, Altrose, Mannose, Gulose, Idose, Talose und Sucrose und/oder deren Derivate, z.B. Etherderivate, Aminoderivate und/oder Acetylderivate wie acetylierte Glucose, z.B. Tetraacetylglucose, Pentaacetylglucose und/oder 2-Acetamido-2-desoxyglucose. Bevorzugte Zuckerbausteine sind Glucose, Fructose, Galactose, Allose, Lactose, Arabinose und Sucrose; Glucose, Galactose und Lactose sind besonders bevorzugt;
   - Onsäuren, insbesondere Gluconsäure, Glucuronsäure;
   - Polyole, wie z.B. Glucamine, Glycerin, Mono- oder Diglyceride, 2-Ethyl-1,3-hexandiol, 2-Hydroxymethylpropantriol, Glycole wie Ethylenglykol, Diethylenglykol, Triethylenglykol, Propylenglykol, Dipropylenglykol, 1,3-Butandiol;
   - Polyhydroxysäuren, wie z.B. Pentahydroxyhexansäure, Tetrahydroxypentansäure und/oder deren Derivate, wie z.B. Ether, Ester und/oder Amide, z.B. Pentahydroxyhexansäureamid und/oder deren physiologisch verträglichen Salzen; weitere Beispiele: Zitronensäure, Äpfelsäure oder Weinsäure.
• Pantolacton
• Panthenol und/oder dessen Derivate;
• weitere Vitamine, wie z.B. Vitamin B6, C und/oder E und/oder deren Derivate;
• Hydroxysäuren, wie z.B. α-, β-Hydroxyfettsäuren bzw. Ketofettsäuren und/oder deren physiologisch verträglichen Salzen; wie beispielsweise Salicylsäure oder Milchsäure, Glyoxylsäure, Glycolsäure.
• wasserlösliche Polymere festigender Wirkung, z.B. Polyvinylpyrrolidon, Vinylacetat/Crotonsäure-Copolymere,
• Antischuppenwirkstoffe wie z.B. Picrotone Olamine, Zink Omadine,
• Wirkstoffe wie Allantoin, Pyrrolidoncarbonsäuren, Pflanzenextrakte,
• pH-Einstell- und Puffermittel wie z.B. Citronensäure/Natriumcitrat, Ammoniumcarbonat, Ammoniumhydrogecarbonat, Guanidincarbonat, Phosphate,
• Komplexbildner wie EDTA, NTA, Organophosphonsäuren, Dipicolinsäure
• Lichtschutzmittel (UV-Absorber)
• Öl-, Fett- und Wachskomponenten, bevorzugt in emulgierter Form,
• Farbstoffe, Trübungs- und Perlglanzmittel sowie
• gegebenenfalls Aerosol-Treibgase

Ein zweiter Gegenstand der Erfindung ist die Verwendung mindestens einer konditionierend wirkenden Verbindung, ausgewählt aus kationischen Polymeren, quaternären Ammoniumverbindungen, Silikonen und Proteinhydrolysaten, in einer wäßrigen Zusammensetzung (A) und gegebenenfalls (B) in einem Verfahren gemäß des ersten Gegenstandes der Erfindung. Dabei werden die im ersten Gegenstand der Erfindung beschriebenen kationischen Polymere bevorzugt verwendet.

Die folgenden Beispiele sollen den Gegenstand der Erfindung näher erläutern:

### Beispiele

**Tabelle 1: Glättungscremes**

| | **Glättungscreme Nr.** | | | | |
|---|---|---|---|---|---|
| **Rohstoff** | **G1 [Gew.%]** | **G2 [Gew.%]** | **G3 [Gew.%]** | **G-V1 [Gew.%]** | **G-V2 [Gew.%]** |
| 1,2-Propylenglykol | 2,00 | 1.00 | - | 2.00 | - |
| Cetyl-/Stearylalkohol ¹ | 9.00 | 9.00 | 9.00 | 9.00 | 9.00 |
| Lanette^{®} E ² | 0.50 | 0.50 | 0.50 | 0.50 | 0.50 |
| Brij^{®} 35 P ³ | 0.50 | 0.50 | 0.50 | 0.50 | 0.50 |
| Natrosol^{®} 250 HR ⁴ | 0.25 | 0.25 | 0.25 | 0.25 | 0.25 |
| Ammoniak (25 %ige wäßrige Lösung) | 5.00 | 3.00 | 1.00 | 5.00 | 1.00 |
| Turpinal^{®} SL ⁵ | 0.25 | 0.25 | 0.25 | 0.25 | 0.25 |
| Ammoniumthioglykolat (71%ige wäßrige Lösung) | 18.00 | 13.00 | 8.00 | 18.00 | 8.00 |
| Ammoniumbicarbonat | 4.00 | 0.30 | - | 4.00 | - |
| Promois^{®} Silk 1000 ⁶ | 1.00 | - | 1.00 | - | - |
| Promois^{®} S 720 ⁷ | - | 0.50 | - | - | - |
| Dow Corning^{®} 1403 fluid ⁸ | 0.50 | 1.00 | 2.00 | - | - |
| Parfüm | 1.00 | 1.00 | 1.00 | 1.00 | 1.00 |
| Wasser | ad 100 | ad 100 | ad 100 | ad 100 | ad 100 |

| | | | | | |
|---|---|---|---|---|---|
| ¹ Gemisch aus 50 Gew.% Cetyl- und 50 Gew.% Stearylalkohol ² INCI-Bezeichnung: Sodium Cetearyl Sulfate (Cognis) ³ Polyethylenglykolmonolaurylether Dodecanol mit 23 Moläquivalent Ethylenoxid (INCI-Bezeichnung: Laureth-23) (Uniquema) ⁴ Hydroxyethylcellulose (Hercules) ⁵ 1-Hydroxyethan-1,1-diphosphonsäure (INCI-Bezeichnung: Etidronic Acid, Aqua (Water)) (Solutia) ⁶ Collagenhydrolysat (INCI-Bezeichnung: Hydrolized Silk) (RITA Corp.) ⁷ Collagenhydrolysat (INCI-Bezeichnung: Hydrolized Silk) (RITA Corp.) ⁸ INCI-Bezeichnung: Dimethicone, Dimethiconol (Dow Corning) | | | | | |

Die Glättungscremes als Zusammensetzung (A) des erfindungsgemäßen Verfahrens tragen die Nummern G1, G2 und G3. Die nicht erfindungsgemäßen Glättungscremes tragen die Nummer G-V1 und G-V2.

**Tabelle 2: Fixiermittel**

| | **Fixiermittel Nr.** | | | |
|---|---|---|---|---|
| **Rohstoff** | **F1 [Gew.%]** | **F2 [Gew.%]** | **F3 [Gew.%]** | **F-V1 [Gew.%]** |
| Cetearylalkohol | 4.00 | 4.00 | 4.00 | 4.00 |
| Eumulgin^{®} B3 ⁹ | 0.50 | 0.50 | 0.50 | 0.50 |
| Ammoniak (25 %ige wäßrige Lösung) | 0.80 | 0.80 | 0.80 | 0.80 |
| Dipicolinsäure | 0.10 | 0.10 | 0.10 | 0.10 |
| Turpinal^{®} SL ⁵ | 1.70 | 1.70 | 1.70 | 1.70 |
| Rheocare^{®} CTH(E) ¹⁰ | 1.00 | 1.00 | - | - |
| Genamin^{®} KDMP¹¹ | - | 2.00 | - | - |
| Merquat^{®} 100 ¹² | - | - | 0.20 | - |
| Wasserstoffperoxid (50 %ige wäßrige Lösung) | 4.00 | 4.00 | 4.00 | 4.00 |
| Wasser | ad 100 | ad 100 | ad 100 | ad 100 |

| | | | | |
|---|---|---|---|---|
| ⁹ Cetylstearylalkohol, ethoxyliert mit 30 Einheiten Ethylenoxid (INCI-Bezeichnung: Ceteareth-30) (Cognis) ¹⁰ Trimethylammonioethyl methacrylat Chlorid Homopolymer, (INCI-Bezeichnung: Polyquaternium-37, Propylene Glycol Dicaprylate/Dicaprate, PPG-1 Trideceth-6) (CRL Cosmetic Rheologies, Ltd.) ¹¹ enthält 85 Gew.% C₂₀₋₂₂-Alkyltrimethylammoniumchlorid als Aktivsubstanz in Isopropanol als Lösemittel, (INCI-Bezeichnung: Behentrimonium Chloride) (Clariant) ¹² Poly(dimethyldiallylammoniumchlorid) (INCI-Bezeichnung: Polyquaternium-6) (Nalco) | | | | |

Die Fixiermittel als Zusammensetzung (B) des erfindungsgemäßen Verfahrens tragen die Nummern F1, F2 und F3. Das nicht erfindungsgemäße Fixiermittel trägt die Nummer F-V1.

### Versuchsdurchführung der Haarglättung

In den beiden untenstehenden Methoden A und B wurden jeweils 30 cm lange und 2.8 g schwere Haarsträhnen aus naturkrausem, unbehandelten Haar südamerikanischen Ursprungs der Firma De Meo Brothers, New York, verwendet.

In einem Zwischen- und Nachbehandlungsschritt wurde eine Spülung gemäß Tabelle 3 in den Methoden A und B verwendet.

**Tabelle 3: Spülung**

| **Rohstoff** | **Menge in Gew.%** |
|---|---|
| Dehyquart^{®} F 75¹³ | 2.50 |
| Rewoquat^{®} W 575 PG¹⁴ | 3.00 |
| Dehyquart^{®} A-CA ¹⁵ | 8.00 |
| Cetyl-/Stearylalkohol ¹ | 8.00 |
| Glyzerinmonostearat | 0.50 |
| Isopropylmyristat | 3.00 |
| Ajidew^{®} NL-50 ¹⁶ | 0.50 |
| p-Hydroxybenzoesäurepropylester | 0.15 |
| p-Hydroxybenzoesäuremethylester | 0.15 |
| 2-Phenoxyethanol | 0.80 |
| Paraffinöl | 3.00 |
| Dow Corning^{®} 1403 fluid⁸ | 0.75 |
| Salcare^{®} SC 96 ¹⁷ | 0.20 |
| Polymer JR 400¹⁸ | 0.50 |
| Tegoamid^{®} S 18 ¹⁹ | 2.00 |
| Nikotinsäureamid | 0.20 |
| D-Panthenol 75 W²⁰ | 0.50 |
| Zitronensäure | 0.35 |
| Wasser | ad 100 |

| | |
|---|---|
| ¹³ Fettalkohole-Methyltriethanolammoniummethylsulfatdialkylester-Gemisch (INCI-Bezeichnung: Distearoylethyl Hydroxyethylmonium Methosulfate, Cetearyl Alcohol) (Cognis Deutschland) ¹⁴ 1-Methyl-2-norpalmalkyl-3-palmfettsäureamidoethylimidazolinium-methosulfat, 75% Aktivsubstanz (INCI-Bezeichnung: Quaternium-87, Propylene Glycol) (Goldschmidt) ¹⁵ Trimethylhexadecylammoniumchlorid, 25% Aktivsubstanz (INCI-Bezeichnung: Aqua (Water), Cetrimonium Chloride) (Cognis) ¹⁶ Pyrrolidoncarbonsäure Natrium Salz, 50% Aktivsubstanz, (INCI-Bezeichnung: Sodium PCA) (Ajinomoto) ¹⁷ Trimethylammonioethyl methacrylat Chlorid Homopolymer, (INCl-Bezeichnung: Polyquaternium-37, Propylene Glycol Dicaprylate/Dicaprate, PPG-1 Trideceth-6) (CIBA) ¹⁸ quaternierte Hydroxyethylcellulose (INCI-Bezeichnung: Polyquaternium-10) (Amerchol) ¹⁹ N,N-Dimethyl-N'-stearoyl-1,3-diaminopropan (INCI-Bezeichnung: Stearamidopropyl Dimethylamine) (Degussa) 20 D-Panthenylalkohol, 75% Aktivsubstanz (BASF) | |

### Methode A

A1) Die Haarsträhne wurde mit einer handelsüblichen, ammoniakhaltigen Oxidationshaarfarbe unter Verwendung von Wasserstoffperoxid als Oxidationsmittel gefärbt. Die Haare wurden so dann mit einem handelüblichen Shampoo gewaschen und mit dem Handtuch frottiert.
A2) Die Haarsträhne wurde gekämmt und 4.8 g einer Glättungscreme (gemäß Tabelle 1 bzw. 4) mit einem Pinsel aufgetragen.
A3) Nach einer Einwirkzeit Z1 von 20 Minuten wurde die Glättungscreme gründlich mit Wasser abgespült.
A4) 1.2 g der Spülung gemäß Tabelle 3 wurde auf das Haar aufgetragen und nach einer Einwirkzeit von einer Minute gründlich mit Wasser ausgespült.
A5) Die Haarsträhne wurde mit heißer Luft unter Zuhilfenahme eines Föns getrocknet, bis die Haare einzeln fielen und nicht mehr durch die Feuchtigkeit zusammenhafteten.
A6) Die Strähne wurde anschließend mittels auf 180°C temperierter Platten des Geräts "Ceramic Flat-Master" (Firma Efalock, Deutschland) mechanisch geglättet. Zu diesem Zweck wurde die Haarsträhne fünf Mal zwischen den Platten des Geräts hindurchgezogen. Mit den Platten wurde dabei ein leichter Anpreßdruck auf das Haar ausgeübt.
A7) Anschließend wurde 5.3 g eines Fixiermittels (gemäß Tabelle 2 bzw. 4) auf die Strähne aufgetragen und nach 15 Minuten Einwirkzeit Z2 wieder ausgespült.
A8) Abschließend wurde die Strähne mit 1.2 g der Spülung gemäß Tabelle 3 behandelt und nach einer Einwirkzeit von 15 Minuten wieder abgespült.

### Methode B

B1) Die Haarsträhne wurde mit einem handelsüblichen Shampoo gewaschen und mit einem Handtuch frottiert.
B2) wie A2)
B3) Nach einer Einwirkzeit Z1 von 30 Minuten wurde die Glättungscreme gründlich mit Wasser abgespült.
B4) wie A4)
B5) wie A5)
B6) wie A6)
B7) wie A7)
B8) wie A8)

Es wurden folgende Methoden mit folgenden Glättungscreme-Fixiermittel-Kombinationen durchgeführt:

**Tabelle 4:**

| | **Glättung (Versuchsnr.)** | | | | |
|---|---|---|---|---|---|
| | **1** | **2** | **3** | **4** | **5** |
| Methode | B | B | A | B | A |
| Glättungscreme (gemäß Tabelle 1) | G1 | G2 | G3 | G-V1 | G-V2 |
| Fixiermittel (gemäß Tabelle 2) | F1 | F2 | F3 | F-V1 | F-V1 |

Die erfindungsgemäßen Glättungsverfahren tragen gemäß Tabelle 4 die Versuchsnummern 1, 2 und 3. Die Glättungsverfahren aus Tabelle 4 mit den Versuchsnummern 4 und 5 sind nicht erfindungsgemäß und wurden zum Vergleich durchgeführt.

Die Haare sind nach Verlauf der erfindungsgemäßen Verfahren gegenüber den nicht erfindungsgemäßen Verfahren einwandfrei und gleichmäßig geglättet und darüber hinaus gut gepflegt. Die Verfahren mit den Versuchsnummern 1, 2 und 3 gemäß Tabelle 4 liefern zusätzlich ein Haar, das nicht statisch aufgeladen ist, während die Haare gemäß der nicht erfindungsgemäßen Glättungsverfahren eine nennenswerte statische Aufladung besitzen.

## Patentansprüche

1. Verfahren zur Glättung keratinhaltiger Fasern, insbesondere menschlicher Haare,
**dadurch gekennzeichnet, daß**
(i) eine wäßrige Zusammensetzung (A) enthaltend mindestens eine keratinreduzierende Verbindung auf die Fasern aufgetragen wird,
(ii) die wäßrige Zusammensetzung (A) nach einer Einwirkzeit Z1 wieder abgespült wird, dann eine wäßrige Zusammensetzung (C), enthaltend mindestens eine konditionierend wirkende Verbindung, ausgewählt aus kationischen Polymeren, quaternären Ammoniumverbindungen, Silikonen und Proteinhydrolysaten, auf die Fasern aufgetragen wird und nach einer Einwirkzeit Z3 wieder abgespült wird,
(iii) die Fasern getrocknet und anschließend
(iv) die Fasern einer Wärmebehandlung unter mechanischer Glättung der Faser bei einer Temperatur von 120-220°C unterworfen werden,
(v) anschließend eine wäßrige Zusammensetzung (B), enthaltend mindestens ein Oxidationsmittel auf die Fasern aufgetragen und
(vi) nach einer Einwirkzeit Z2 wieder abgespült wird,
wobei die wäßrige Zusammensetzung (A) als konditionierend wirkende Verbindungen mindestens ein Proteinhydrolysat und mindestens ein Silikon und gegebenenfalls mindestens eine weitere konditionierend wirkende Verbindung, ausgewählt aus kationischen Polymeren und quaternären Ammoniumverbindungen enthält, und wobei die wäßrige Zusammensetzung (B) gegebenenfalls mindestens eine weitere konditionierend wirkende Verbindung, ausgewählt aus kationischen Polymeren, quaternären Ammoniumverbindungen, Silikonen und Proteinhydrolysaten, enthält.

2. Verfahren nach Anspruch 1 **dadurch gekennzeichnet, daß** in der wäßrigen Zusammensetzung (A) die keratinreduzierende Verbindungen in einer Menge von 5-15 Gew.-%, bezogen auf die gesamte Zusammensetzung (A), enthalten sind.

3. Verfahren nach einem der Ansprüche 1 oder 2, **dadurch gekennzeichnet, daß** in der wäßrigen Zusammensetzung (A) zusätzlich mindestens ein anionisches, nichtionisches, amphoteres oder zwitterionisches Tensid enthalten ist.

4. Verfahren nach einem der Ansprüche 1 bis 3, **dadurch gekennzeichnet, daß** die wäßrige Zusammensetzung (B) als konditionierend wirkende Verbindung mindestens ein kationisches Polymer enthält.

5. Verfahren nach einem der Ansprüche 1 bis 4, **dadurch gekennzeichnet, daß** die quaternären Ammoniumverbindungen ausgewählt sind aus Alkyltrimethylammoniumhalogeniden, Dialkyldimethylammoniumhalogeniden, Trialkylmethylammoniumhalogeniden und Esterquats.

6. Verfahren nach einem der Ansprüche 1 bis 5, **dadurch gekennzeichnet, daß** die Silikone ausgewählt sind aus linearen, cyclischen oder verzweigten Silikonen ausgewählt aus den Cyclomethiconen, Dimethiconen, Dimethiconolen, Dimethiconcopolyolen, Amodimethiconen, Trimethylsilylamodimethiconen und Phenyltrimethiconen.

7. Verfahren nach einem der Ansprüche 1 bis 6, **dadurch gekennzeichnet, daß** die Proteinhydrolysate ausgewählt sind, aus Elastin-, Kollagen-, Keratin-, Seiden- und Milcheiweiß-Proteinhydrolysaten.

8. Verfahren nach einem der Ansprüche 1 bis 7, **dadurch gekennzeichnet, daß** das kationische Polymer mindestens ein Strukturelement der Formel (I) enthält, 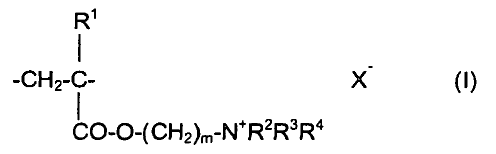 worin
• R¹ für ein Wasserstoffatom oder eine Methylgruppe steht,
• R², R³ und R⁴ unabhängig voneinander für eine C₁-C₄-Alkylgruppe, C₁-C₄-Alkenylgruppe oder C₁-C₄-Hydroxyalkylgruppe stehen
• m = 1, 2, 3 oder 4 ist und
o X ein physiologisch verträgliches organisches oder anorganisches Anion ist.

9. Verfahren nach Anspruch 8, **dadurch gekennzeichnet, daß** das kationische Polymer ein Homopolymer ist.

10. Verfahren nach einem der Ansprüche 1 bis 9, **dadurch gekennzeichnet, daß** in den wäßrigen Zusammensetzungen (A) und/oder (B) zusätzlich mindestens eine viskositätserhöhende Verbindung enthalten ist.

11. Verfahren nach einem der Ansprüche 1 bis 10, **dadurch gekennzeichnet, daß** die wäßrige Zusammensetzung (A) eine Viskosität von 5000-50000 mPa·s, insbesondere von 8000-20000 mPa·s, bei 20°C (gemessen mit einem Brookfield-Viskometer, Spindel Nr. 6 bei 20 rpm) besitzt.

12. Verfahren nach einem der Ansprüche 1 bis 11, **dadurch gekennzeichnet, daß** die keratinhaltigen Fasern vor dem Schritt (i) angefeuchtet werden.

13. Verfahren nach einem der Ansprüche 1 bis 12, **dadurch gekennzeichnet, daß** direkt nach dem Schritt (i) und/oder während der Einwirkzeit Z1 aus Schritt (ii) und gegebenenfalls direkt vor Schritt (iv) die Fasern mechanisch, insbesondere durch Kämmen, geglättet werden.

14. Verfahren nach einem der Ansprüche 1 bis 13, **dadurch gekennzeichnet, daß** die Wärmebehandlung mittels entsprechend temperierter Platten erfolgt, in dem die Platte auf die zu glättende Faser gedrückt wird und die an die Faser gedrückte Platte entlang der Faser bewegt wird.

15. Verfahren nach Anspruch 14, **dadurch gekennzeichnet, daß** die keratinhaltige Faser zwischen zwei entsprechend temperierte Platten gepreßt wird und die Platten zugleich entlang der Faser bewegt werden.

16. Verfahren nach Anspruch 1, **dadurch gekennzeichnet, daß** die wäßrige Zusammensetzung (C) als konditionierend wirkende Verbindung ein kationisches Polymer gemäß Formel (I) aus Anspruch 8 enthält.

17. Verwendung mindestens einer konditionierend wirkenden Verbindung, ausgewählt aus kationischen Polymeren, quaternären Ammoniumverbindungen, Silikonen und Proteinhydrolysaten, in einer wäßrigen Zusammensetzung (A) und gegebenenfalls eine wäßrigen Zusammensetzung (B) in einem Verfahren gemäß einem der Ansprüche 1 bis 16.

## Claims

1. Method for smoothing fibres containing keratin, in particular human hair, **characterized in that**
(i) an aqueous composition (A) comprising at least one keratin-reducing compound is applied to the fibres,
(ii) the aqueous composition (A) is rinsed off again after a contact time T1, then an aqueous composition (C) comprising at least one conditioning compound selected from cationic polymers, quaternary ammonium compounds, silicones and protein hydrolysates is applied to the fibres and rinsed off again after a contact time T3,
(iii) the fibres are dried and then
(iv) the fibres are subjected to a heat treatment with mechanical smoothing of the fibres at a temperature of 120-220°C,
(v) then an aqueous composition (B) comprising at least one oxidizing agent is applied to the fibres and
(vi) is rinsed off again after a contact time T2,
where the aqueous composition (A) comprises, as conditioning compounds, at least one proteinhydrolysate and at least one silicone and optionally at least one further conditioning compound selected from cationic polymers and quaternary ammonium compounds, and where the aqueous composition (B) optionally comprises at least one further conditioning compound selected from cationic polymers, quaternary ammonium compounds, silicones and protein hydrolysates.

2. Method according to Claim 1, **characterized in that**, in the aqueous composition (A), the keratin-reducing compounds are present in an amount of 5-15% by weight, based on the total composition (A).

3. Method according to one of Claims 1 or 2, **characterized in that**, in the aqueous composition (A), at least one anionic, nonionic, amphoteric or zwitterionic surfactant is additionally present.

4. Method according to one of Claims 1 to 3, **characterized in that**, the aqueous composition (B) comprises at least one cationic polymer as conditioning compound.

5. Method according to one of Claims 1 to 4, **characterized in that**, the quaternary ammonium compounds are selected from alkyltrimethylammonium halides, dialkyldimethylammonium halides, trialkylmethylammonium halides and ester quats.

6. Method according to one of Claims 1 to 5, **characterized in that** the silicones are selected from linear, cyclic or branched silicones selected from the cyclomethicones, dimethicones, dimethiconols, dimethicone copolyols, amodimethicones, trimethylsilylamodimethicones and phenyltrimethicones.

7. Method according to one of Claims 1 to 6, **characterized in that** the protein hydrolysates are selected from the protein hydrolysates of elastin, collagen, keratin, silk and milk protein.

8. Method according to one of Claims 1 to 7, **characterized in that** the cationic polymer contains at least one structural element of the formula (I), 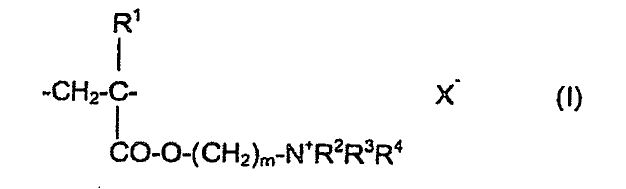 in which
• R¹ is a hydrogen atom or a methyl group,
• R², R³ and R⁴, independently of one another, are a C₁-C₄-alkyl group, C₁-C₄-alkenyl group or C₁-C₄-hydroxyalkyl group,
• m = 1, 2, 3 or 4 and
• X⁻ is a physiologically compatible organic or inorganic anion.

9. Method according to Claim 8, **characterized in that** the cationic polymer is a homopolymer.

10. Method according to one of Claims 1 to 9, **characterized in that**, in the aqueous compositions (A) and/or (B), at least one viscosity-increasing compound is additionally present.

11. Method according to one of Claims 1 to 10, **characterized in that** the aqueous composition (A) has a viscosity of 5000-50 000 mPa·s, in particular 8000-20 000 mPa·s, at 20°C (measured using a Brookfield viscometer, spindle No. 6 at 20 rpm).

12. Method according to one of Claims 1 to 11, **characterized in that** the fibres containing keratin are wetted before step (i).

13. Method according to one of Claims 1 to 12, **characterized in that** directly after step (i) and/or during the contact time T1 from step (ii) and optionally directly before step (iv), the fibres are smoothed mechanically, in particular by combing.

14. Method according to one of Claims 1 to 13, **characterized in that** the heat treatment takes place by means of appropriately heated plates by pressing the plate onto the fibre to be smoothed and moving the plate pressed onto the fibre along the fibre.

15. Method according to Claim 14, **characterized in that** the fibre containing keratin is pressed between two appropriately heated plates and the plates are at the same time moved along the fibre.

16. Method according to Claim 1, **characterized in that** the aqueous composition (C) comprises a cationic polymer according to formula (I) from Claim 8 as conditioning compound.

17. Use of at least one conditioning compound selected from cationic polymers, quaternary ammonium compounds, silicones and protein hydrolysates in an aqueous composition (A) and optionally an aqueous composition (B) in a method according to one of Claims 1 to 16.

## Revendications

1. Procédé pour lisser les fibres kératiniques, en particulier les cheveux humains, **caractérisé en ce que**,
(i) une composition aqueuse (A) contenant au moins un composé réducteur de kératine est appliquée sur les fibres,
(ii) la composition aqueuse (A) après une durée d'action Z1 est éliminée de nouveau par rinçage, puis une composition aqueuse (C) contenant au moins un composé à effet de conditionnement, choisi parmi les polymères cationiques, les composés ammonium quaternaires, les silicones et les hydrolysats de protéines, est appliquée sur les fibres et éliminée de nouveau par rinçage après une durée d'action Z3,
(iii) les fibres sont séchées et ensuite
(iv) les fibres sont soumises à un traitement thermique avec lissage mécanique de la fibre à une température de 120-220°C,
(v) ensuite une composition aqueuse (B), contenant au moins un agent d'oxydation, est appliquée sur les fibres, et
(vi) éliminée de nouveau par rinçage après une durée d'action Z2,
la composition aqueuse (A) contenant en tant que composés à effet de conditionnement, au moins un hydrolysat de protéines et au moins une silicone, et éventuellement au moins un composé supplémentaire à effet de conditionnement, choisi parmi les polymères cationiques et les composés ammonium quaternaires, et la composition aqueuse (B) contenant éventuellement au moins un composé supplémentaire à effet de conditionnement, choisi parmi les polymères cationiques, les composés ammonium quaternaires, les silicones et les hydrolysats de protéines.

2. Procédé selon la revendication 1, **caractérisé en ce que**, dans la composition aqueuse (A), les composés réducteurs de kératine sont contenus en une quantité de 5 à 15% en poids, par rapport à la composition totale (A).

3. Procédé selon l'une quelconque des revendications 1 ou 2, **caractérisé en ce qu'**au moins un tensio-actif anionique, non ionique, amphotère, ou zwitterionique est contenu en outre dans la composition aqueuse (A).

4. Procédé selon l'une quelconque des revendications 1 à 3, **caractérisé en ce que** la composition aqueuse (B) contient en tant que composés à effet de conditionnement, au moins un polymère cationique.

5. Procédé selon l'une quelconque des revendications 1 à 4, **caractérisé en ce que** les composés ammonium quaternaires sont choisis parmi les halogénures d'alkyltriméthylammonium, les halogénures de dialkyldiméthylammonium, les halogénures de trialkylméthylammonium et les esterquats.

6. Procédé selon l'une quelconque des revendications 1 à 5, **caractérisé en ce que** les silicones sont choisies parmi les silicones linéaires, cycliques ou ramifiés, choisies parmi les cyclométhicones, les diméthicones, les diméthiconols, les diméthicone-copolyols, les amodiméthicones, les triméthylsilylamodiméthicones, et les phényltriméthicones.

7. Procédé selon l'une quelconque des revendications 1 à 6, **caractérisé en ce que** les hydrolysats de protéines sont choisis parmi les hydrolysats de protéines d'élastine, de collagène, de kératine, de soie et d'albumine lactique.

8. Procédé selon l'une quelconque des revendications 1 à 7, **caractérisé en ce que**, le polymère cationique contient au moins un élément structural de formule (I), 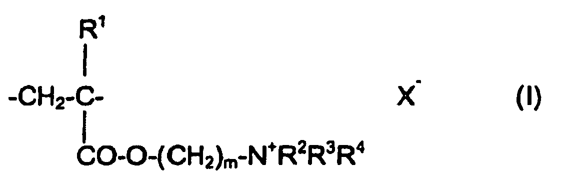 dans laquelle
• R¹ représente un atome d'hydrogène ou un groupe méthyle,
• R², R³ et R⁴ représentent indépendamment l'un de l'autre un groupe alkyle en C₁ à C₄, un groupe alcényle en C₁ à C₄ ou un groupe hydroxyalkyle en C₁ à C₄
• m=1, 2, 3 ou 4 et
• X⁻ est un anion organique ou inorganique acceptable sur le plan physiologique.

9. Procédé selon la revendication 8, **caractérisé en ce que** le polymère cationique est un homopolymère.

10. Procédé selon l'une quelconque des revendications 1 à 9, **caractérisé en ce qu'**au moins un composé augmentant la viscosité est contenu en outre dans les compositions aqueuses (A) et/ou (B).

11. Procédé selon l'une quelconque des revendications 1 à 10, **caractérisé en ce que** la composition aqueuse (A) possède une viscosité de 5 000 à 50 000 mPa.s, en particulier de 8 000 à 20 000 mPa.s, à 20°C (mesurée par un viscosimètre Brookfield, broche N°6 à 20 tr/mn).

12. Procédé selon l'une quelconque des revendications 1 à 11, **caractérisé en ce que** les fibres kératiniques sont humidifiées avant l'étape (i).

13. Procédé selon l'une quelconque des revendications 1 à 12, **caractérisé en ce que** les fibres sont lissées mécaniquement, en particulier par peignage directement après l'étape (i) et/ou pendant la durée d'action Z1 de l'étape (ii) et éventuellement directement avant l'étape (iv).

14. Procédé selon l'une quelconque des revendications 1 à 13, **caractérisé en ce que** le traitement thermique se réalise au moyen de plaques mises à température de façon appropriée, procédé dans lequel la plaque est pressée sur les fibres à lisser, et la plaque pressée sur les fibres est déplacée le long de la fibre.

15. Procédé selon la revendication 14, **caractérisé en ce que** la fibre kératinique est pressée entre deux plaques mises à température de façon appropriée et les plaques sont déplacées en même temps le long de la fibre.

16. Procédé selon la revendication 1, **caractérisé en ce que** la composition aqueuse (C) contient en tant que composé à effet de conditionnement, un polymère cationique selon la formule (I) de la revendication 8.

17. Utilisation d'au moins un composé à effet de conditionnement, choisi parmi les polymères cationiques, les composés ammonium quaternaires, les silicones, et les hydrolysats de protéines, dans une composition aqueuse (A) et éventuellement une composition aqueuse (B) dans un procédé selon l'une quelconque des revendications 1 à 6.
